# EUROPEAN PATENT APPLICATION

(11) **EP 1 393 706 A1**
(43) Date of publication of application: **03.03.2004**
(21) Application number: 02255677.3
(22) Date of filing: 14.08.2002
(51) Int. Cl.: A61K 7/00, B01J 13/22, B01J 13/06, A61K 9/50

(54) **Fragranced compositions comprising encapsulated material**

(71) Applicant: QUEST INTERNATIONAL B.V., 1411 GP Naarden (NL)
(72) Inventor: The designation of the inventor has not yet been filed
(74) Representative: Matthews, Heather Clare

(57) **Abstract**

A fragranced composition such as a water-based consumer product comprises material (e.g. perfume) encapsulated within shell capsules, each capsule comprising an encapsulating wall having an inner surface and an outer surface, with a coating on the inner surface and/or outer surface of the shell wall, the composition further comprising surfactant and/or solvent. The coating can improve the barrier properties of the shell and can enhance retention of the encapsulated materials within the shell.

## Description

### Field of the Invention

This invention concerns compositions, particularly consumer products, containing encapsulated material, and relates to such compositions and encapsulates.

### Background to the Invention

It is known to encapsulate perfume or other materials, in small capsules (or micro-capsules), typically having a diameter less than 1000 microns, for a variety of reasons relating to the protection, delivery and release of the perfume or other material. One type of capsule, referred to as a wall or shell capsule, comprises a generally spherical hollow shell of e.g. perfume-insoluble material, typically polymer material, within which perfume or other material is contained.

If such capsules are incorporated in certain solvent and/or surfactant-containing consumer products, e.g. shampoos, stability problems can arise, with the encapsulated material tending to leach out of the capsules into the product over time, at least in cases where the capsule contents are soluble in ingredients of the product.

The present invention aims to address such stability problems.

### Summary of the Invention

In one aspect the present invention provides a fragranced composition comprising material encapsulated within shell capsules, each capsule comprising an encapsulating wall having an inner surface and an outer surface, with a coating on the inner surface and/or outer surface of the shell wall; and surfactant and/or solvent.

The term "fragranced composition" means a composition containing at least 0.1% by weight of one or more perfume materials (as discussed below). The perfume material may be present in the composition, encapsulated within shell capsules and/or in unencapsulated form.

Preferably, the composition is a product, particularly a consumer product, conveniently in liquid or solid form. Preferably, the product is a liquid product and more preferably a water-based product.

Examples of products include fabric care products such as fabric detergents e.g. laundry liquids and laundry powders, fabric conditioners e.g. rinse conditioners and sheet conditioners, fabric treatment products including fabric refresher products, e.g. sprays, starch sprays, ironing sprays and stain remover sprays; personal care products such as skin care, hair care and personal cleansing products including hair shampoos, body washes and shower gels, bath foams, toilet soap, toothpaste, mouthwash, deodorants and antiperspirants, skin creams and lotions and the like, colognes, body sprays, personal perfumes; and household products such as toilet cleaners, hard surface cleaners, abrasive cleaners, general purpose cleaners and bleaches.

Typically the product will be of generally conventional composition, as is known to those skilled in the art, and may comprise further excipients appropriate to the nature of the product. Suitable excipients may include fixatives, softening agents, enzymes, builders, bleaching agents, bleach activators, suspending agents, thickeners, silicones, emollients, humectants, vitamins, flavours, perfumes, antibacterial agents, etc. Details of suitable excipients for products such as fabric detergents, fabric conditioners, personal cleansing products, particularly hair shampoos and shower gels, and household cleaners are described in WO 98/28396 and WO 98/28398. Details of suitable excipients for 'leave-on' products such as deodorants and antiperspirants, skin creams, colognes etc, are described, for example, in L. Appell, "The Formulation and Preparation of Cosmetics Fragrances and Flavours", Micelle Press (1994), Chapter 1.

The encapsulated material generally comprises a first material which is conveniently at least partially, preferably substantially and more preferably completely soluble, in the surfactant and/or solvent of the compositions defined herein. Preferably, the first material is substantially water-insoluble to facilitate preparation of an emulsion or dispersion of the material prior to encapsulation.

Suitable types of such first materials which may be encapsulated within shell capsules as defined herein include perfumes, moisturisers and conditioning agents, sunscreening agents, insecticides, dental flavours, insect repellents, antimicrobial agents, deodorant actives and physiological coolants, or mixtures thereof.

As is well known, a perfume normally consists of a mixture of a number of perfume materials, each of which has an odour or fragrance. The number of perfume materials in a perfume is typically 10 or more. The range of fragrant materials used in perfumery is very wide; the materials come from a variety of chemical classes, but in general are water-insoluble oils. In many instances, the molecular weight of a perfume material is in excess of 150, but does not exceed 300.

Perfumes used in the present invention can be mixtures of conventional perfume materials. Such materials are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic, and heterocyclic compounds.

Such perfume materials are mentioned, for example, in S. Arctander, Perfume and Flavor Chemicals (Montclair, N.J., 1969), in S. Arctander, Perfume and Flavor Materials of Natural Origin (Elizabeth, N.J., 1960) and in "Flavor and Fragrance Materials - 1991", Allured Publishing Co. Wheaton, I11. USA.

Examples of perfume materials which can be used in the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nonpol, nopyl acetate, 2-phenyl-ethanol, 2-penylethyl acetate, benzyl alcohol, benzyl acetate, benzyl salicylate, styrallyl acetate, benzyl benzoate, amyl salicylate, dimethylbenzyl-carbinol, trichloromethylphenyl-carbinyl acetate, p-tert-butylcyclohexyl acetate, isononyl acetate, vetiveryl acetate, vetiverol, α-hexylcinnamaldehyde, 2-methyl-3-(p-tert-butylpheyl)propanal, 2-methyl-3-(p-isopropylphenyl)propanal, 2-(p-tert-butylpheyl)-propanal, 2,4-dimethyl-cyclohex-3-enyl-carboxaldehyde, tricyclodecenyl acetate, tricyclodecenyl propionate,4-(4-hydroxy-4-methylpentyl)-3-cyclohexenecarboxyaldehyde, 4-(4-methyl-3-pentenyl)-3-cyclohexenecarboxaldehyde, 4-acetoxy-3-pentyltetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethyl-acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate.

The perfume should be substantially free of water-miscible materials such as dipropylene glycol. Solvents which can be used for perfumes include, for example: diethyl phthalate, triethyl citrate, etc.

Examples of suitable moisturisers and conditioning agents useful herein include cyclic silicones and esters such as isopropyl myristate. See also WO 96/12468 page 56.

Examples of sunscreening agents useful herein include octyl methoxy cinnamate available for example as Parsol MCX (Parsol MCX is a Trade Mark) from Hoffman LaRoche, nonylmethoxycinnamate, 2-ethyl hexyl salicylate, benzophenone, and para amino benzoic acid and its esters. See also WO 96/12468 page 55 to 56.

Examples of suitable insecticides useful herein include pyrethroid compounds such as pyrethrum and 3-phenoxybenzyl 2,2-dimethyl-3-12',2'-dichlorovinyl)-cyclopropane-carboxylate (permethrin).

A wide range of well known dental flavours may be used which may be natural or synthetic, e.g. oil of spearmint, peppermint, wintergreen, sassafras, clove, sage, eucalyptus, marjoram, cinnamon, lemon and orange, menthol, eugenol, aniseed, cinnamic aldehyde, menthyl acetate and methyl salicylate.

As examples of suitable insect repellents may be mentioned N,N-diethyl-m-toluamide (DEET), benzamide, methyl neodecanamide, hexane-1,2-diol, methyl and 3-(N-butylacetylamino)-propionic acid ethyl ester, commercially available as Merck IR 3535 from Merck & Co. Inc.

Preferably, the antimicrobial agent is water-insoluble. An example of a suitable water-insoluble antimicrobial agent for use herein is 5-chloro-2-[(2,4-dichlorophenyl)oxy]phenol, more commonly known as Triclosan (Triclosan is a Trade Mark), commercially available for example as Irgasan DP300 (Irgasan DP300 is a Trade Mark) from Ciba Speciality Chemicals.

Suitable deodorant actives include, for example, materials such quaternary ammonium compounds e.g. cetyl-trimethylammonium bromide, lauroyl sarcosine, N-myristoyl glycine, diaminoalkyl amides such as L-lysine hexadecyl amide, zinc citrate and zinc pyrithione.

Examples of suitable physiological coolants useful herein include menthol, menthol PCA, menthyl lactate, menthyl succinate and menthyl carbonate.

The encapsulated material may comprise one or more first materials.

In addition to the above-described first material of the shell capsules, or in the alternative, there may also be present one or more second materials which may be insoluble in the surfactant and/or solvent of the compositions defined herein. Examples of such insoluble materials include certain conditioning agents (e.g. high molecular weight dimethicones), pigments (e.g. titanium dioxide) and anti-dandruff agents (e.g. zinc pyrethione).

Optional further materials which may be encapsulated in the shell capsules herein include dyes and preservatives etc.

In a preferred embodiment, the encapsulated material is a perfume composition, which typically comprises at least 80% and preferably at least 90% by weight of the total weight of the perfume composition of perfume materials having an octanol-water partition coefficient of greater than 2.5 (in logarithmic form to base 10), and typically less than 35% and preferably less than 20% by weight of the total weight of the perfume composition of perfume materials having a octanol-water partition coefficient of greater than 5 (in logarithmic form to base 10).

The octanol-water partition coefficient (P) of a material i.e. the ratio of a material's equilibrium concentration in octanol and water, is well known in the literature as a measure of hydrophobicity and water solubility (see Hansch and Leo, Chemical Reviews, 526 to 616, (1971), 71; Hansch, Quinlan and Lawrence, J. Organic Chemistry, 347 to 350 (1968), 33). High partition coefficient values are more conveniently given in the form of their logarithm to the base 10, log P. While log P values can be measured experimentally i.e. directly, and measured log P data is available for many perfumes, log P values are most conveniently calculated or approximately estimated using mathematical algorithms. There are several recognised calculation or estimation methods available commercially and/or described in the literature (see for example A Leo, Chem.Rev 93(4), 1281-1306, (1993), "Calculating log P oct from structures"). Generally these models correlate highly but may for specific materials produce log P values which differ in absolute terms (by up to 0.5 log units or even more). However, no one model is universally accepted as the most accurate across all compounds. This is particularly true for estimates on materials of high log P (say 4 or greater). In the present specification, log P values are obtained using the estimation software commercially available as 'LogP' from Toronto-based Advanced Chemistry Development Inc (ACD) which is well-known to the scientific community, and accepted as providing high-quality predictions of log P values. References to log P values thus mean values obtained using the ACD software.

The percentage by weight of perfume materials referred to herein is relative to the total weight of perfume materials present in the composition and excludes, for example, the presence of any optional solvents or diluents etc, such as e.g. diethyl phthalate, dipropylene glycol, benzyl benzoate, acetyle tributyl citrate, triethyl citrate, Hercolyn D and isopropyl myristate.

Preferred perfume materials for a perfume composition useful herein, are therefore those perfume materials having an octanol-water partition coefficient (log P) falling within the range 3 to 5. Examples of such perfume materials include the following:

1-(o-tert-butylcyclohexyloxy)butan-2-ol, 1,4-dimethylcyclohexane carboxylic acid methyl ester, 10-iso-propyl-2,7-dimethyl-1-oxaspiro[4.5]3,6-decadiene, 1-methyl-3-(2-methylpropyl)cyclohexanol, 3-(4-tert-butylphenyl)-2-methylpropanal, 4-methyl-3-decen-5-ol, 7,9-dimethylspiro[5,5] undecan-3-one, acetaldehyde ethyl cis-3-hexenyl acetal, acetyl diisoamylene (Q), alcohol C9 (nonanol), aldehyde C9 (nonanal), aldehyde C10 (decanal), aldehyde C11 (undecylenic aldehyde), alicate (Q), allyl caproate, allyl cyclohexyl propionate, allyl heptylate, allyl hexanoate, amyl benzoate, amyl cinnamic alcohol, amyl cinnamic aldehyde, amyl salicylate, amyl valerate, anethole, anther (Q), aurantion (Q), bangalol (Q), beauvertate (Q), benzophenone, benzyl cinnamate, benzyl phenylacetate, benzyl salicylate, borneol, bourgeonal (Q), butyl phenylacetate, calyxol (Q), carvacrol, carvacryl ethyl ether, carveol, cervolide (Q), cineole, Cis-hex-3-enyl salicylate, cistulate (Q), citral, citral dimethyl acetal, citrathal (Q), citronellal, citronellol, citronellyl acetate, citronellyl nitrile, citronellyl oxyacetaldehyde, citronellyl propionate, cressanther (Q), cumin nitrile, cyclamen aldehyde (Q), cyclohexyl salicylate, damascone alpha, dec-4-enal, dec-9-enal, dec-9-enol, dihydroanethole, dihydrocarveol, dihydrocarvone, dihydrojasmone, dihydrolinalol, dihydromyrcenol, dihydromyrcenyl acetate, dihydroterpineol, dihydroterpinyl acetate, dimethylheptanol, diphenyl oxide, diphenylmethane, dodecyl nitrile, dupical (Q), elintaal (Q), empetaal (Q), ethyl cinnamate, ethyl heptylate, ethyl linalol, ethyl nonanoate, ethyl octanoate, ethyl safranate (Q), fenchyl acetate, fenchyl alcohol, florocyclene (Q), frutonile (Q), gardocylene (Q), geraniol, geranyl acetate, geranyl nitrile, geranyl propionate, gyrane (Q), herbanate (Q), hexyl benzoate, hexyl cinnamic aldehyde, hexyl salicylate, inonyl acetate, inonyl propionate, ionones, iso E super (IFF), isoamyl salicylate, isobergamate (Q), isoborneol, isobornyl acetate, isobutyl benzoate, isobutyl cinnamate, isoeugenol, isojasmone, jasmatone (Q), jasmopyran (Q), jessate (Q), kerfoline (Q), lime oxide, linalool, linalyl acetate, linalyl propionate, maceal (Q), mefrosol (Q), menthanyl acetate, menthol, menthyl acetate, methyl chavicol, methyl eugenol, methyl ionones, methyl isoeugenol, methyl nonyl acetaldehyde, methyl octine carbonate, methyl salicylate, musk R1 (Q), myrcene, neobergamate (Q), nerol, nerolin, neryl acetate, nopyl acetate, octyl acetate, orange terpenes, ortholate (Q), para cresyl phenyl acetate, para-tert-butylcyclohexanol, para-tert-butylcyclohexanyl acetate, pelargene (Q), petiole (Q), phenyl benzoate, phenyl ethyl methyl ethyl carbinol, phenylethyl isobutyrate, phenylethyl methyl ethyl carbinol, phenylethyl phenylacetate, phenylethyl salicylate, pinenes, pivacyclene (Q), rhubafuran (Q), rose oxide, roseacetone, rosyrane (Q), terpinyl acetate, tetrahydrogeraniol, tetrahydrogeranyl acetate, tetrahydrolinalol, tetrahydrolinalyl acetate, tetrahydromyrcenol, thymol, undecanal, undecen-2-nitrile, yara yara.

Materials identified by (Q) above are trade marks or trivial names, and available from Quest International. Materials identified by (IFF) above are trade marks or trivial names, and are available from International Flavours and Fragrances.

The shell capsules may be prepared using a range of conventional methods known to those skilled in the art for making shell capsules such as coacervation, interfacial polymerisation and polycondensation.

The process of coacervation typically involves encapsulation of a generally water-insoluble material by the precipitation of colloidal material(s) onto the surface of droplets of the material. Coacervation may be simple e.g. using one colloid such as gelatin, or complex where two or possibly more colloids of opposite charge, such as gelatin and gum arabic or gelatin and carboxymethyl cellulose, are used under carefully controlled conditions of pH, temperature and concentration. Coacervation techniques are described, e.g. in US2800458, US2800457, GB929403, EP385534 and EP376385.

Interfacial polymerisation produces encapsulated shells from the reaction of at least one oil-soluble wall forming material present in the oil phase with at least one water-soluble wall forming material present in the aqueous phase. A polymerisation reaction between the two wall-forming materials occurs resulting in the formation of covalent bonds at the interface of the oil and aqueous phases to form the capsule wall. An example of a shell capsule produced by this method is a polyurethane capsule.

Polycondensation involves forming a dispersion or emulsion of water-insoluble material e.g. perfume in an aqueous solution of precondensate of polymeric materials under appropriate conditions of agitation to produce capsules of a desired size, and adjusting the reaction conditions to cause condensation of the precondensate by acid catalysis, resulting in the condensate separating from solution and surrounding the dispersed water-insoluble material fill to produce a coherent film and the desired micro-capsules. Polycondensation techniques are described, e.g. in US3516941, US4520142, US4528226, US4681806, US4145184 and GB2073132.

A preferred method for forming shell capsules useful herein is polycondensation, typically to produce aminoplast encapsulates comprising a shell of urea-formaldehyde or melamine-formaldehyde polymer.

Thus, preferably, the shell capsules are aminoplast capsules.

By modifying process conditions shell capsules of a desired size can be produced in known manner. The shell capsules typically have a diameter in the range 1 to 500 microns, preferably 5 to 300 microns, more preferably 5 to 50 microns. Preferred sizes for the shell capsules will depend upon their intended use. For example, shell capsules employed in a shampoo preferably have a diameter in the range 1 to 10 microns. If necessary, the shell capsules as initially produced may be filtered or screened to produce a product of greater size uniformity.

The shell capsules comprise a coating on the inner surface, the outer surface, or both the inner and outer surfaces of the shell wall. In general, the shell of the capsules useful herein can be considered as made up of a very tight collection of strands of polymer(s) which generally provides a barrier to the encapsulated material. It is thought however, that when the shell capsules are mixed with a surfactant-containing and/or solvent-containing composition e.g. consumer product, the surfactant and/or solvent present in the composition swells the shell and sufficiently separates the strands of polymer to produce pores through which the encapsulated material may pass. This is a particular problem when the encapsulated material is to some extent surfactant and/or solvent soluble e.g. perfume, as typically, the encapsulated material will be lost to the composition over a relatively short period of time. It has been found by the present inventors, however, that the application of a coating to the inner surface and/or outer surface of the shell wall improves the barrier properties of the shell and thus may enhance retention of the encapsulated materials in surfactant-containing and/or solvent-containing compositions. Without wishing to be bound or limited by theory, it is thought that the coating stiffens the shell to prevent it swelling when in contact with surfactant and/or solvent and/or the coating blocks any pores created when the shell is swollen. A coating may be partial or complete. Preferably, the one or more coatings are complete, so that the shell capsules are thus preferably substantially impermeable.

The application of a coating to the inner surface of the shell capsules may be carried out by a number of methods. One approach involves the use of a suitable material for the coating which is insoluble in the material to be encapsulated, but can be dissolved in a water-soluble solvent e.g. ethanol, carbitol etc., which is miscible with the material to be encapsulated. The approach involves dissolving the coating material typically a polymer, in the solvent and then dissolving this mixture in the material to be encapsulated. The material to be encapsulated is then emulsified into e.g. a standard aminoplast capsule forming solution. As the emulsion forms, the solvent is lost to the water and precipitates out at the surface of the emulsion droplets. The normal encapsulation process is then carried out and the coating deposited on the inner surface of the shell.

In a further approach, a material is used that is immiscible with both the material to be encapsulated and water, and is capable of forming a thin film at the water interface. An example of such a material is a silicone. If this material e.g. silicone is a liquid, a shell encapsulate comprising a coating of silicone on the inner surface of the shell can be prepared by dispersing the material to be encapsulated within the silicone and then emulsifying this mixture so that an emulsion is formed where droplets of encapsulated material are surrounded by a thin film of silicone. The encapsulation process is then carried out as normal. Alternatively, a thin film may be formed at the surface by dispersing the material to be encapsulated in water, adding the second material e.g. silicone and allowing it to coat the encapsulating material droplets subsequently.

Typically, water-insoluble film-forming polymers comprise the inner surface coating. Preferably, such polymers are insoluble in the encapsulated material but soluble in a solvent that is miscible with the material to be encapsulated. Suitable examples include; ethylene-maleic anhydride, polyamine, waxes e.g. carbowax, polyvinylpyrrolidone (PVP) and its co-polymers such as polyvinylpyrrolidone-ethyl acrylate (PVP-EA), polyvinylpyrrolidone-vinyl acrylate, polyvinylpyrrolidone methylacrylate (PVP-MA), polyvinylpyrrolidone/vinyl acetate, polyvinyl acetal, polyvinyl butyral, polysiloxane, propylene maleic anhydride, maleic anhydride derivatives and co-polymers of the above, e.g. polyvinyl methyl ether/maleic anhydride. Preferably, the inner wall coating comprises polysiloxane, PVP or PVP co-polymers, and more preferably PVP co-polymers, particularly polyvinylpyrrolidone-methyl acrylate or polyvinylpyrrolidone-ethyl acrylate.

A coating may be applied to the outer surface of a shell capsule by a variety of conventional coating techniques including spraying, fluid bed coating, precipitating etc. For example a coating, typically of polymer, may be precipitated at elevated temperature onto the surface of the capsule. The shell capsule to be coated is thus formed in a separate step, prior to the application of the coating to the outer surface of the shell wall. Depending on the composition of the outer surface coating, a coated shell capsule may be prepared for example, by coacevation or polycondensation.

The outer surface coating typically comprises high molecular weight, film-forming polymers, which may optionally be crosslinked. By "high molecular weight" is meant a molecular weight of greater than 5000. The polymer is preferably water soluble. Generally, low molecular weight water-soluble materials (e.g. short chain saccharides) are not suitable. Non-limiting examples of suitable polymers include; polyvinyl alcohol, styrene-butadiene latex, gelatin, gum arabic, carboxymethyl cellulose, carboxymethyl hydroxyethyl cellulose, hydroxyethyl cellulose, other modified celluloses, sodium alginate, chitosan, casein, pectin, resorcinol, modified starch, polyvinyl acetal, polyvinyl butyral, polyvinyl methyl ether/maleic anhydride, polyvinyl pyrrolidone (PVP) and its co-polymers (e.g. polyvinylpyrrolidone/vinyl acetate (PVP/VA)), melamine-formaldehyde and urea-formaldehyde.

Preferably, the outer surface of the shell is coated with polyvinyl alcohol or a PVP copolymer. Particularly preferred is a coating of a polyvinyl alcohol having a high hydrolysis level and high molecular weight. Typically the polyvinyl alcohol is 85 to 100% hydrolysed, preferably at least 90% hydrolysed, and more preferably at least 96% hydrolysed e.g. 96 to 99% hydrolysed.

The coating (inner and/or outer) may be cross-linked in known manner, e.g. by interfacial cross-linking.

A shell capsule useful herein may comprise more than one coating on the outer surface of the shell.

Coated shell capsules typically have a wall thickness in the range 0.03 to 30 microns. The wall thickness may be regulated and controlled according to the encapsulate size and by varying the relative preparations of coating and shell polymer.

Typically, the weight ratio of polymer shell wall material to encapsulated material is in the range 1:10 to 3:2 and preferably in the range 1:10 to 1:2. The coating on the inner surface and/or outer surface will increase these weight ratios.

The solvent of the composition may be selected from those well known to those skilled in the art, and include propylene glycol, dipropylene glycol, diethylene glycol monoethyl ether, isopropyl myristate, ethanol, isopropyl alcohol, butyl alcohol, diethylene glycol monobutyl ether, glycerin, dipropylene glycol mono-n-butyl ether, orange oil and mineral oil.

The surfactant of the composition may be selected from those well known to those skilled in the art, including anionic surfactants, nonionic surfactants, cationic surfactants, amphoteric surfactants, zwitterionic surfactants and mixtures thereof. Appropriate surfactant(s) will depend on the nature of the composition, as discussed above, as is known to those skilled in the art. Anionic surfactants include soaps, synthetic detergent surfactants, sulphate surfactants, sulphonate surfactants and N-acylamino surfactants. Nonionic surfactants include alkyl phenol ethoxylates, polyethylene glycol/polypropylene glycol block copolymers, fatty alcohol and fatty acid ethoxylates, long chain tertiary amine oxides, long chain tertiary phosphine oxides, long chain dialkyl sulphoxides, alkyl polysaccharides and polyethylene glycol glyceryl fatty esters. For further details of surfactants see, e.g., WO 96/12468 page 8 to 21.

The weight ratio of solvent/surfactant: capsules in the composition is conveniently in the range 100:1 to 5:1.

As discussed above, the coated shell capsules described herein, possess improved impermeability properties in surfactant-containing and/or solvent-containing compositions compared with the uncoated shell encapsulates of the prior art. Thus, advantageously, when shell capsules comprising a coating on the inner surface and/or outer surface of the shell are incorporated in surfactant-containing and/or solvent-containing compositions, the coated shell capsules demonstrate improved retention of the encapsulated material and thus demonstrate improved in-storage stability. Coated shell capsules comprising encapsulated material such as perfume, when incorporated in a shampoo, typically prevent perfume from leaching from the encapsulates for weeks and possibly months. By contrast, perfume-containing uncoated shell capsules of the prior art tend to leach perfume from the encapsulates over days, e.g. at 45°C.

In a further aspect, the present invention provides capsules comprising encapsulated perfume, the perfume being encapsulated within an aminoplast capsule which comprises a coating of polyvinyl alcohol on the outer surface of the shell.

The invention will be further described, by way of illustration, in the following examples.

### Examples

The following formulations are used in the Examples.

**Perfume A** - this perfume is used in all the capsule examples below and is supplied by Quest International under the code HW 4180B.

**Shampoo Base B** - the following hair shampoo formulation is used in Comparative Examples 1, 2 and 4 below:

| Ingredient | % by weight |
|---|---|
| Sodium lauryl ether (2 mole) sulphate | 9.6 |
| Ammonium luaryl ether (2 mole) sulphate | 4.5 |
| Sodium chloride | 2.0 |
| Citric acid | q.s. to give pH 6.0-6.5 |
| Preservative | q.s. |
| Water | balance to 100% |

**Rinse Conditioner Base C -** the following rinse conditioner formulation is used in Comparative Example 3 below:

| Ingredient | % by weight |
|---|---|
| Rewoquat WE18 * | 16.7 |
| CaCl₂ (10% aq) | 0.5 |
| Formaldehyde | 0.1 |
| Distilled water | to 100.0 |

| | |
|---|---|
| *ex Goldschmidt (a division of Degussa AG) | |

### Preparation

The water is heated to 50°C. Separately, Rewoquat is heated to 50°C and then slowly dispersed into the water and stirred until cool. Formaldehyde is then added and mixed well. The calcium chloride solution is slowly added and mixed for five minutes.

### Reference Example 1

The following example illustrates the formation of a standard polycondensation microcapsule based on melamine/formaldehyde polymer. A solution of 66g of a 10 % solution of ethyelene maleic anhydride was added to 130g of distilled water and adjusted to a pH of 4.5 using a 20% solution of sodium hydroxide. Separately, a 40% solution of melamine in 37% formaldehyde was prepared. 100g of perfume A (the "Internal Phase") were then added to the anhydride solution, which was maintained under continuous agitation to allow the formation of an emulsion of the perfume in water. 72g of the melamine formaldehyde solution were then added. The emulsion was heated to 55°C in a water bath and held there for two hours under continuous agitation and allowed to cool overnight. The capsules produced by this method had a mean particle size of 8µm with a perfume content of 27% in the capsule slurry.

### Example 1

This example illustrates the formation of a coating of polyvinyl pyrrolidone on the inner surface of a capsule wall. 10g of polyvinylpyrrolidone (Luviskol K-90, ex BASF) were dissolved in 90g of isopropyl alcohol. 12g of this resulting solution was then dissolved in 88g of perfume A. The resultant mixture was then agitated and warmed to 35°C for eight hours to form a clear homogeneous solution. This solution was then used as the Internal Phase to produce capsules by the method of Reference Example 1. The resultant capsules had a mean particle size of 6µm with a perfume content of 24.2% in the capsule slurry.

### Example 2

This example illustrates the formation of a coating of silicone fluid on the inner surface of a capsule wall. 390g of perfume A was added to a laboratory mixing vessel and mixed with 24g of polysiloxane fluid (Dow Corning Silicone DC200/100). This mixture was then emulsified with a Silverson mixer for one hour. 100g of the resultant emulsion was then used as the Internal Phase to produce capsules by the method of Reference Example 1. The resultant capsules had a mean particle size of 10µm with a perfume content of 25.6% in the capsule slurry.

### Example 3

The following example illustrates the formation of a polyvinylpyrrolidone (PVP) coating on the inner surface of a capsule wall, further strengthened by interfacial crosslinking. Capsules were prepared using the formulation illustrated in Example 1. However, the encapsulation process was modified by the inclusion of 0.3g of a crosslinking agent, toluene diisocyanate, in the PVP pre-solution. The capsules produced in this example had a mean particle size of 4µm with a perfume content of 24.3 % in the capsule slurry.

### Comparative Example 1

This example illustrates the enhanced perfume retention properties of capsules according to one approach of the invention (having a coating on the inner surface of a capsule wall) compared to standard capsules. The capsule slurries of Reference Example 1 and Examples 1-3 were mixed into unperfumed shampoo base B to produce the following formulations, each containing 0.15wt% perfume (all expressed as percent by weight):

| Shampoo Code | BR1 | B1 | B2 | B3 |
|---|---|---|---|---|
| Shampoo base B | 99.444 | 99.380 | 99.414 | 99.383 |
| Reference Example 1 | 0.556 | - | - | - |
| Example 1 | - | 0.620 | - | - |
| Example 2 | - | - | 0.586 | - |
| Example 3 | - | - | - | 0.617 |

The shampoos were then stored in glass jars for one month at 37°C and examined by transmission light microscopy to determine the degree of perfume loss from the capsules. The capsules in shampoo BR1 were found to be completely empty whilst the capsules in shampoos B1, B2 and B3 were all found to contain significant levels of perfume, thus illustrating the utility of the invention.

### Example 4

This example illustrates the formation of a coating of polyvinyl alcohol on the outer surface of a capsule wall using capsules of reference Example 1. Perfume containing microcapsules were prepared using the method outlined in Reference Example 1. At the completion of the 2 hours at 55°C, the microcapsule emulsion was heated to 80°C.

Simultaneously, a 20% solution of fully hydrolysed polyvinyl alcohol in water was prepared and heated to 80°C. 60g of the polyvinyl alcohol solution were added to the warmed microcapsule slurry and agitation continued for 24 hours during which time the mixture cools slowly. The polyvinyl alcohol precipitates onto the microcapsules thus forming a coating. The final microcapsule slurry had a perfume content of 23.4%.

### Example 5

This example illustrates the formation of a coating of polyvinyl acetal on the outer surface of a capsule wall using capsules of reference example 1. Perfume containing microcapsules are prepared using the method outlined in Reference Example 1. At the completion of the 2 hours at 55°C, the microcapsule emulsion was heated to 80°C. Simultaneously, a 20% solution of fully hydrolysed polyvinyl alcohol in water was prepared and heated to 80°C. 60g of the polyvinyl alcohol solution were added to the warmed microcapsule slurry. The pH was then adjusted to 4.5 using sulphuric acid and 6g of 37% formaldehyde was added to the mixture. Agitation was continued for 24 hours during which time the mixture cools slowly. During this time, polyvinyl acetal is formed and precipitates onto the microcapsules forming a coating. The final microcapsule slurry had a perfume content of 23.0%.

### Example 6

This example illustrates the formation of a coating of a water-insoluble polyvinyl pyrrolidone/vinyl acetate copolymer on the outer surface of a capsule wall using reference example 1. Perfume containing microcapsules are prepared using the method outlined in Reference Example 1. At the completion of the 2 hours at 55°C, the microcapsule emulsion is maintained at 55°C. Simultaneously, a 10% solution of polyvinyl pyrrolidone/vinyl acetate copolymer in isopropyl alcohol was prepared and heated to 55°C. 40g of the polyvinyl pyrrolidone/vinyl acetate copolymer solution were added to the warmed microcapsule slurry and agitation was continued for 4 hours at 55°C. The mixture was then allowed to cool under agitation for a further 24 hours. The polyvinyl pyrrolidone/vinyl acetate copolymer precipitates onto the microcapsules as the isopropyl alcohol evaporates from the mixture. The final microcapsule slurry contained 26.8% perfume.

### Example 7

This example illustrates the formation of a coating of carboxymethyl cellulose (CMC) on the outer surface of a capsule wall using capsules of reference example 1. A 10% aqueous solution of carboxymethyl cellulose (Blanose Gum, ex ISP Chemicals) was prepared by mixing and heating until a clear homogeneous solution was formed. Meanwhile, the capsule slurry of Reference Example 1 was spray-dried to form a free-flowing powder using a laboratory spray dryer (Mobile Minor, supplied by Niro). The resultant powder was then charged into a laboratory fluid bed dryer (Aeromatic AG, supplied by Niro) and the capsules were coated by spraying of the CMC solution using standard fluid bed coating techniques to achieve a coating thickness of 0.4µm. The resultant capsules were analysed and found to contain 79% perfume.

### Comparative Example 2

This example illustrates the enhanced perfume retention properties of capsules according to another approach of the invention (having a coating on the outer surface of a capsule wall) compared to standard capsules. The capsule slurries of Reference Example 1 and Examples 4-7 were mixed into unperfumed shampoo base B to produce the following formulations, each containing 0.2wt% perfume (all expressed as percent by weight):

| Shampoo Code | BR2 | B4 | B5 | B6 | B7 |
|---|---|---|---|---|---|
| Shampoo base B | 99.259 | 99.145 | 99.130 | 99.254 | 99.747 |
| Reference Example 1 | 0.741 | - | - | - | - |
| Example 4 | - | 0.855 | - | - | - |
| Example 5 | - | - | 0.870 | - | - |
| Example 6 | - | - | - | 0.746 | - |
| Example 7 | - | - | - | - | 0.253 |

The shampoos were then stored in glass jars for one month at 37°C and examined by transmission light microscopy to determine the degree of perfume loss from the capsules. The capsules in shampoo BR2 were found to be completely empty whilst the capsules in shampoos B4, B5, B6 and B7 were all found to contain significant levels of perfume, thus illustrating the utility of the invention.

### Comparative Example 3

This example illustrates the enhanced perfume retention properties of capsules according to the invention compared to standard capsules. The capsule slurries of Reference Example 1 and Examples 1, 4, 6 and 7 were mixed into unperfumed rinse conditioner base C to produce the following formulations, each containing 0.2wt% perfume (all expressed as percent by weight):

| Conditioner Code | CR1 | C1 | C4 | C6 | C7 |
|---|---|---|---|---|---|
| Conditioner base C | 99.259 | 99.174 | 99.145 | 99.254 | 99.747 |
| Reference Example 1 | 0.741 | - | - | - | - |
| Example 1 | - | 0.826 | - | - | - |
| Example 4 | - | - | 0.855 | - | - |
| Example 6 | - | - | - | 0.746 | - |
| Example 7 | - | - | - | - | 0.253 |

The rinse conditioners were then stored in glass jars for one month at 37°C and examined by transmission light microscopy to determine the degree of perfume loss from the capsules. The capsules in conditioner CR1 were found to be completely empty whilst the capsules in conditioners C1, C4, C6 and C7 were all found to contain significant levels of perfume, thus illustrating the utility of the invention.

### Reference Example 2

This example illustrates the formation of a typical complex coacervate encapsulate. 25g of acid-treated gelatin (isoelectric point 8, gel strength 180g Bloom) and 2.5g of carboxymethyl cellulose (Blanose Gum, ex ISP chemicals) were added to 700g water and mixed with heating at 60°C until a clear homogeneous solution was formed. The pH was then adjusted to 5.5 with a 5% aqueous solution of sodium hydroxide and then cooled to 10°C. 100g of perfume A was then added to this solution whilst mixing was maintained to generate an emulsion of perfume. 25g of 10% aqueous formaldehyde was then mixed into the emulsion and left to stand for 15 minutes. The pH of the emulsion was then adjusted to 10 by dropwise addition of a 10% aqueous solution of NaOH, causing the formation of hardened microcapsules. The resulting slurry was analysed and found to contain 11.7 % perfume by weight.

### Example 8

This example illustrates the formation of a coating of polyvinyl alcohol on the outer surface of a capsule using capsules of reference example 2. Perfume containing microcapsules are prepared using the method outlined in Reference Example 2. At the completion of hardening of the capsule walls, the microcapsule emulsion was heated to 80°C. Simultaneously, a 20% solution of fully hydrolysed polyvinyl alcohol in water was prepared and heated to 80°C. 60g of the polyvinyl alcohol solution were then added to the warmed microcapsule slurry and agitation continued for 24 hours during which time the mixture cooled slowly. The polyvinyl alcohol precipitates onto the microcapsules thus forming a coating. The microcapsule slurry was measured to have a final perfume content of 11.0%.

### Example 9

This example illustrates the formation of a coating of polyvinyl acetal on the outer surface of a capsule using capsules of reference example 2. Perfume containing microcapsules are prepared using the method outlined in Reference Example 2. At the completion of hardening of the capsule walls, the microcapsule emulsion was heated to 80°C. In parallel, a 20% solution of fully hydrolysed polyvinyl alcohol in water was prepared and heated to 80°C. 60g of the polyvinyl alcohol solution was added to the warmed microcapsule slurry and pH adjusted to 4.5 using sulphuric acid. 6g of 37% formaldehyde were then added to the mixture. Agitation was continued for 24 hours during which time the mixture cools slowly. The polyvinyl acetal is formed and precipitates onto the microcapsules. The final microcapsule slurry contained 10.8% perfume.

### Example 10

This example illustrates the formation of a coating of polyvinyl pyrrolidone/vinyl acetate copolymer on the outer surface of a capsule. Perfume containing microcapsules are prepared using the method outlined in Reference Example 2. At the completion of hardening of the capsule walls, the microcapsule emulsion was heated to 55°C. A 10% solution of polyvinyl pyrrolidone/vinyl acetate copolymer in isopropyl alcohol was prepared and heated to 55°C. 40g of the polyvinyl pyrrolidone/vinyl acetate copolymer solution were added to the warmed microcapsule slurry and agitation continued for 4 hours at 55°C. The mixture was then allowed to cool under agitation for a further 24 hours. The polyvinyl pyrrolidone/vinyl acetate copolymer was precipitated onto the microcapsules as the isopropyl alcohol evaporates from the mixture. The measure perfume level in the capsule slurry was 11.6%.

### Example 11

This example illustrates the formation of a coating of carboxymethyl cellulose (CMC) onto the outer surface of a capsule using capsules of reference example 2. A 10% aqueous solution of carboxymethyl cellulose (Blanose Gum, ex ISP Chemicals) was prepared by mixing and heating until a clear homogeneous solution was formed. Meanwhile, the capsule slurry of Reference Example 2 was spray-dried to form a free-flowing powder using a laboratory spray dryer (Mobile Minor, supplied by Niro). The resultant powder was then charged into a laboratory fluid bed dryer (Aeromatic AG, supplied by Niro) and the capsules were coated by spraying of the CMC solution using standard fluid bed coating techniques to achieve a coating thickness of 0.6µm. The resultant capsules were analysed and found to contain 16% perfume.

### Comparative Example 4

This example illustrates the enhanced perfume retention properties of capsules according to the approach of the invention (having a coating on the outer surface of a capsule wall) compared to standard capsules. The capsule slurries of Reference Example 2 and Examples 8-10 were mixed into unperfumed shampoo base B to produce the following formulations (all expressed as percent by weight):

| Shampoo Code | BR2 | B8 | B9 | B10 |
|---|---|---|---|---|
| Shampoo base B | 98.29 | 98.18 | 98.15 | 98.28 |
| Reference Example 2 | 1.71 | - | - | - |
| Example 8 | - | 1.82 | - | - |
| Example 9 | - | - | 1.85 | - |
| Example 10 | - | - | - | 1.72 |

The shampoos were then stored in glass jars for one week at 37°C and examined by transmission light microscopy to determine the degree of perfume loss from the capsules. The capsules in shampoo BR2 were found to be completely empty whilst the capsules in shampoos B8, B9 and B10 were found to contain significant levels of perfume, thus illustrating the utility of the invention.

## Claims

1. A fragranced composition comprising material encapsulated within shell capsules, each capsule comprising an encapsulating wall having an inner surface and an outer surface, with a coating on the inner surface and/or outer surface of the shell wall; and surfactant and/or solvent.

2. A composition according to claim 1, wherein the composition is a product, particularly a consumer product.

3. A composition according to claim 2, wherein the product is a water-based product.

4. A composition according to any one of the preceding claims, wherein the encapsulated material comprises a first material which is at least partially, preferably substantially and more preferably completely soluble, in the surfactant and/or solvent of the composition.

5. A composition according to claim 4, wherein the first material is a perfume.

6. A composition according to claim 5, wherein the perfume is in the form of a perfume composition, which comprises at least 80% and preferably at least 90% by weight of the total weight of the perfume composition of perfume materials having an octanol-water partition coefficient of greater than 2.5 (in logarithmic form to base 10).

7. A composition according to claim 6, wherein less than 35%, and preferably less than 20%, by weight of the total weight of the perfume composition comprises perfume materials having a octanol-water partition coefficient of greater than 5 (in logarithmic form to base 10).

8. A composition according to any one of the preceding claims, wherein the shell capsules are prepared by coacervation, interfacial polymerisation or polycondensation.

9. A composition according to claim 8, wherein the shell capsules are aminoplast capsules.

10. A composition according to any one of the preceding claims, wherein the shell capsules have a diameter in the range 1 to 500 microns, preferably 5 to 300 microns, more preferably 5 to 50 microns.

11. A composition according to any one of the preceding claims, wherein the inner surface of the shell wall is coated with a water-insoluble film-forming polymer

12. A composition according to claim 11, wherein the polymer is selected from: ethylene-maleic anhydride, polyamine, waxes e.g. carbowax, polyvinylpyrrolidone (PVP) and its co-polymers such as polyvinylpyrrolidone-ethyl acrylate (PVP-EA), polyvinylpyrrolidone-vinyl acrylate, polyvinylpyrrolidone methylacrylate (PVP-MA), polyvinylpyrrolidone/vinyl acetate, polyvinyl acetal, polyvinyl butyral, polysiloxane, propylene maleic anhydride, maleic anhydride derivatives and co-polymers of the above, e.g. polyvinyl methyl ether/maleic anhydride.

13. A composition according to any one of the preceding claims, wherein the outer surface of the shell wall is coated with a high molecular weight, film-forming polymer, which may optionally be crosslinked.

14. A composition according to claim 13, wherein the polymer is water soluble.

15. A composition according to claim 13 or 14, wherein the polymer is selected from: polyvinyl alcohol, styrene-butadiene latex, gelatin, gum arabic, carboxymethyl cellulose, carboxymethyl hydroxyethyl cellulose, hydroxyethyl cellulose, other modified celluloses, sodium alginate, chitosan, casein, pectin, resorcinol, modified starch, polyvinyl acetal, polyvinyl butyral, polyvinyl methyl ether/maleic anhydride, polyvinyl pyrrolidone (PVP) and its co-polymers (e.g. polyvinylpyrrolidone/vinyl acetate (PVP/VA)), melamine-formaldehyde and urea-formaldehyde.

16. A composition according to any one of the preceding claims, wherein the coated shell capsules have a wall thickness in the range 0.03 to 30 microns.

17. A composition according to any one of the preceding claims, wherein the weight ratio of polymer shell wall material to encapsulated material is in the range 1:10 to 3:2 and preferably in the range 1:10 to 1:2.

18. A composition according to any one of the preceding claims, wherein the weight ratio of solvent/surfactant: capsules in the composition is in the range 100:1 to 5:1.

19. Capsules comprising encapsulated perfume, the perfume being encapsulated within an aminoplast capsule which comprises a coating of polyvinyl alcohol on the outer surface of the shell.
